# EUROPÄISCHE PATENTSCHRIFT

(11) **EP 0 877 602 B1**
(45) Veröffentlichungstag und Bekanntmachung des Hinweises auf die Patenterteilung: **23.01.2002**
(21) Anmeldenummer: 97902186.2
(22) Anmeldetag: 18.01.1997
(51) Int. Cl.: A61K 9/14, C07K 14/785

(54) **VERFAHREN ZUR HERSTELLUNG VON PULVERFÖRMIGEN LUNGENSURFACTANT-ZUBEREITUNGEN**
PROCESS FOR THE PRODUCTION OF POWDERED PULMONARY SURFACTANT PREPARATIONS
PROCEDE POUR FABRIQUER DES PREPARATIONS DE SURFACTANTS PULMONAIRES EN POUDRE

(30) Priorität: 24.01.1996 DE 19602332; 06.03.1996 EP 96103442
(43) Veröffentlichungstag der Anmeldung: 18.11.1998
(73) Patentinhaber: Byk Gulden Lomberg Chemische Fabrik GmbH, 78467 Konstanz (DE)
(72) Erfinder: EISTETTER, Klaus, D-78465 Konstanz (DE)
(86) Internationale Anmeldenummer: EP9700230
(87) Internationale Veröffentlichungsnummer: WO9726863

(56) Entgegenhaltungen:
- EP-A- 0 119 056
- EP-A- 0 655 237
- DATABASE WPI Week 8349 Derwent Publications Ltd., London, GB; AN 83-835368 XP002010938 & JP 58 183 621 A (TEIJIN KK) , 26.Oktober 1983

## Beschreibung

### Technisches Gebiet

Die Erfindung betrifft ein Verfahren zur Herstellung von pulverförmigen Lungensurfactant-Zubereitungen.

### Stand der Technik

Die Lunge aller Wirbeltiere enthält ein Substanzgemisch, das als "Lungensurfactant" bezeichnet wird. Es zeigt oberflächenaktive Eigenschaften und setzt die Oberflächenspannung im Alveolarbereich der Lungen so weit herab, daß ein Kollaps der finalen Atemwegsbereiche bei der Ausatmung vermieden wird. Dieses Substanzgemisch reguliert die Oberflächenspannung dynamisch, so daß der nach dem Laplaceschen Gesetz zu erwartende Kollaps der kleinen Alveolen zugunsten der größeren durch entsprechende Anpassung der Oberflächenspannung vermieden wird. Als Ergebnis entsteht so eine wohl ausbalancierte, histologisch und physiologisch stabile Struktur der Lunge.

Lungensurfactant wird von den alveolären Pneumozyten vom Typ II in Form lamellarer Körperchen (lamellar bodies) sezemiert. Dieses sind kompakte Einheiten aus Phospholipid-Doppelschichten (bilayem) mit einem hohen Anteil an Dipalmitoylphosphatidylcholin (DPPC) und Phosphatidylglycerin (PG). Als weitere essentielle Komponenten sind im Lungensurfactant Proteine enthalten, die mit SP-A, SP-B und SP-C bezeichnet werden. SP-A ist ein hochmolekulares Glycoprotein, das bei der Regulation der Sekretion eine entscheidende Rolle spielt.

Die hydrophoben Proteine SP-C und, in geringerem Maße, SP-B übernehmen bei der Ausbildung des monomolekularen Oberflächenfilms (dem Surfactant im engeren Sinne) die Rolle "thermodynamischer Katalysatoren". Durch die Anwesenheit dieser Proteine wird die Spreitungskinetik enorm beschleunigt. Erst dadurch ist die verzögerungsfreie Anpassung der Surfactant-Zusammensetzung an die jeweiligen Oberflächenspannungserfordernisse möglich. Diese Eigenschaften spiegeln sich in dem extrem hydrophoben Charakter der Proteine, insbesondere des SP-C, wider.

Bei frühgeborenen Babys ist deren Lunge noch nicht oder noch nicht in ausreichendem Maß in der Lage, Lungensurfactant zu produzieren, was zu einem lebensbedrohlichen Sauerstoffmangel führt (Infant Respiratory Distress Syndrome, IRDS). IRDS stellt die Haupttodesursache bei Frühgeborenen dar.

Seit vielen Jahren hat es sich bewährt, IRDS durch Einbringen von Lungensurfactant-Zubereitungen in die Lungen der betroffenen Kinder zu behandeln. Aus Pilotstudien ist bekannt, daß auch bei ARDS (Adult Respiratory Distress Syndrom) einschließlich ALI (Acute Lung Injury) Lungensurfactant-Zubereitungen klinisch wirksam sind.

Lungensurfactant-Zubereitungen können aus tierischen Lungen in einem aufwendigen Extraktions- und Zentrifugationsprozeß (Lungenlavage) gewonnen werden oder aus einzelnen Komponenten zusammengestellt werden.

Die WO 92/06703 beschreibt die Herstellung synthetischer Lungensurfactant-Zubereitungen durch Eindampfen einer Chloroform-Lösung enthaltend Phospholipide, wie Dipalmitoylphosphatidylcholin (DPPC) und Dioleylphosphatidylethanolamin (DOPE), und Cholesterol am Rotationsverdampfer zu einem dünnen Film, der gewünschtenfalls zusammen mit geeigneten Proteinen in einem Puffer resuspendiert wird.

Aus WO 91/00871 ist es bekannt, eine organische Lösung einer Lungensurfactant-Zubereitung, die ein gentechnologisch hergestelltes Lungensurfactant-Protein enthält, einzuengen, mit einem Puffer zu rehydratisieren und anschließend zu lyophilisieren. Das erhaltene Lyophilisat hat den Nachteil, daß es vor der Verabreichung 15 Minuten bei 37°C rehydratisiert werden muß, was vom Anwender als sehr umständlich und fehleranfällig empfunden wird.

In der EP 0119056 wird ein Verfahren zur Herstellung einer Lungensurfactant-Zubereitung angegeben, bei dem alle Bestandteile in einem organischen Lösungsmittel gelöst werden, die erhaltene Lösung unter vermindertem Druck zur Trockne eingeengt wird, der erhaltene Rückstand in einem wäßrigen Medium bei erhöhter Temperatur über einen längeren Zeitraum resuspendiert wird und die erhaltene Suspension einer Gefriertrocknung unterworfen wird. Auch dieses Verfahren ist technisch sehr aufwendig.

Die DE 3229179 offenbart ein Verfahren zur Herstellung einer proteinfreien Lungensurfactant-Zubereitung, bei dem die Bestandteile in Eisessig gelöst werden und die erhaltene Lösung gefriergetrocknet wird. Nachteilig an diesem Verfahren ist die Verwendung von Eisessig, weil dies umfangreiche Sicherheitsmaßnahmen erforderlich macht.

In der EP 0655237 wird vorgeschlagen, Arzneistoffzubereitungen, die in Form eines Suspensionsaerosols inhalativ verabreicht werden sollen, durch Sprühtrocknung aus Ethanol/Wasser-Gemischen herzustellen. Dieses Verfahren wird u. a. als geeignet beschrieben für Zusammensetzungen, die hydrophile Proteine, wie z. B. Ecatibantacetat, Humaninsulin und Buserelinacetat, enthalten.

### Beschreibung der Erfindung

Die der vorliegenden Erfindung zugrundeliegende Aufgabe wird darin gesehen, ein Verfahren zur Herstellung von proteinhaltigen pulverförmigen Lungensurfactant-Zubereitungen enthaltend hydrophobe Lungensurfactant-Proteine anzugeben, das technisch möglichst wenig aufwendig ist und zu einem vorteilhaft anzuwendenden lagerstabilen Produkt führt.

Es wurde nun überraschenderweise festgestellt, daß diese Aufgabe dadurch gelöst werden kann, daß man eine organische Lösung oder Suspension enthaltend hydrophobe Lungensurfactant-Proteine und gewünschtenfalls weitere Bestandteile einer Sprühtrocknung unterwirft.

Man erhält nach diesem Verfahren ein Produkt, das lange Zeit lagerstabil ist und sich ohne besonderen Aufwand vor der Anwendung resuspendieren läßt. Als besonderer Vorteil des erhaltenen Pulvers ist seine geringe Teilchengröße (1 bis 5 µm) hervorzuheben, die eine inhalative Verabreichung erlaubt. Dieser Aspekt ist von besonderer Bedeutung bei der Verwendung von Lungensurfactant-Zubereitungen als Schlepper für über die Lunge applizierbarer Arzneistoffe.

Es ist sehr erstaunlich und bisher nicht erklärbar, wieso die zum Teil sehr temperaturempfindlichen Bestandteile von Lungensurfactants die Bedingungen des Sprühtrocknungsprozesses überstehen. So ist z. B. bekannt, daß das Lungensurfactant-Protein SP-C oberhalb von -20°C sehr rasch aggregiert und damit inaktiviert wird. Den erfindungsgemäßen Sprühtrocknungsprozeß dagegen übersteht dieses Protein ohne merkliche Zersetzung und liegt dann als lockeres Pulver vor, das bei Raumtemperatur lagerfähig ist.

Gegenstand der Erfindung ist daher ein Verfahren, das dadurch gekennzeichnet ist, daß man eine organische Lösung oder Suspension enthaltend hydrophobe Lungensurfactant-Proteine und gewünschtenfalls weitere Bestandteile einer Sprühtrocknung unterwirft.

Weitere Gegenstände ergeben sich aus den Patentansprüchen.

Als hydrophobe Lungensurfactant-Proteine kommen gleichermaßen solche natürlichen Ursprungs als auch synthetisch, einschließlich gentechnologisch, hergestellte Proteine, insbesondere SP-B und SP-C, sowie deren Mischungen in Frage. Unter synthetischen Proteinen sollen auch solche Proteine verstanden werden, deren Aminosäuresequenz von der Aminosäuresequenz natürlich vorkommender Lungensurfactant-Proteine mehr oder weniger stark abweicht, einschließlich solcher synthetische Proteine, die eine vollkommen eigenständig im Hinblick auf ihre Lungensurfactant-Eigenschaft konzipierte Aminosäuresequenz aufweisen, wie sie beispielsweise in EP 0593094 und EP 92/22315 beschrieben sind. Diese Proteine können durch bekannte Verfahren isoliert, synthetisiert und aufgereinigt sein.

Als Lösungsmittel zur Herstellung einer organischen Lösung oder Suspension eignen sich erfindungsgemäß Alkohole, wie Methanol, Ethanol, 1-Propanol, 2-Propanol, Butanole, Chlorkohlenwasserstoffe, wie Dichlormethan, Chloroform etc., Aceton, Ether, Kohlenwasserstoffe, Benzol, Toluol und deren Gemische, wobei auch Wasser enthalten sein kann, soweit eine Mischbarkeit mit Wasser gegeben ist. Der maximale Wassergehalt beträgt 25 Gew.-%. Bevorzugt ist ein Wassergehalt von 5 bis 15 Gew.-%. Dem Fachmann ist es ein leichtes, aufgrund seiner Fachkenntnisse auf dem Gebiet der Sprühtrocknung und nötigenfalls durch übliche Versuche die für die zu trocknenden Surfactantgemische am besten geeigneten Lösungsmittel bzw. Lösungsmittelgemische auszuwählen.

Als weitere Bestandteile enthalten die Lungensurfactant-Zubereitungen die üblichen Stoffe, wie insbesondere Phospholipide, Carbonsäuren und Puffersubstanzen.

Vor Beginn der Sprühtrocknung kann die Lösung durch ein Sterilfilter filtriert werden. Die Sprühtrocknung erfolgt auf an sich bekannte Weise. Eine ausführliche Darstellung dieser Technik findet sich bei K. Masters, Spray Drying Handbook, 5th Ed. 1991, und J. Broadhead, S. K. Edmond Ronan, C. T. Rhodes, The Spray Drying of Pharmaceuticals, Drug Dev. Ind. Pharm. 18, 1169 (1992). Das Prinzip der Sprühtrocknung besteht darin, eine Lösung oder Suspension des zu trocknenden Produkts in feine Tröpfchen zu zerteilen und mit einem heißen Gasstrom zu trocknen. Der nach Verdampfen des Lösungsmittels zurückbleibende Feststoffanteil wird aus dem Gasstrom mittels eines Zyklons und/oder durch eine Filtereinheit abgetrennt und gesammelt.

Erfindungsgemäß hat es sich als zweckmäßig erwiesen, als Lösungsmittel Alkohole und Chlorkohlenwasserstoffe, insbesondere Methanol, Ethanol, 2-Propanol und Chloroform und deren Gemische, gegebenenfalls mit einem geringen Wasserzusatz (bis maximal 25 Gew.-%), zu verwenden. Als Trocknungsgase kommen insbesondere Luft und Stickstoff in Frage.

Als Gaseintrittstemperatur sind 60 bis 200°C, vorzugsweise 90 bis 150°C zweckmäßig. Die Gasaustrittstemperatur wird durch entsprechende Steuerung der Sprühleistung und/oder der Gasmenge auf 40 bis 80°C, vorzugsweise 50 bis 70°C gehalten.

### Herstellungsbeispiele

### Beispiel 1

7,0 g 1,2-Dipalmitoyl-3-sn-phosphatidylcholin, 2,5 g 1-Palmitoyl-2-oleoyl-3-sn-phosphatidylglycerolnatrium, 205 mg Calciumchloriddihydrat und 250 mg Palmitinsäure werden unter Erwärmen auf 60°C in 300 ml Ethanol/Wasser (85:15) gelöst, auf Raumtemperatur abgekühlt und mit 350 ml einer Lösung von SP-C in Chloroform/Methanol 9:1 (c=429 mg/l) gemischt. Die resultierende Lösung wird in einem Laborsprühtrockner Büchi B 191 sprühgetrocknet. Sprühbedingungen: Trocknungsgas Luft, Eintrittstemperatur 90°C, Austrittstemperatur 52 - 54°C. Man erhält ein lockeres Pulver.

### Beispiel 2

Eine Lösung von aus Rinderlungen gewonnenem Surfactant (erhalten durch Extraktion und Reinigungsschritte, wie z. B. beschrieben in EP 406732) in Chloroform/Methanol wird unter folgenden Bedingungen sprühgetrocknet: Laborsprühtrockner Büchi B 191, Trocknungsgas Stickstoff, Eintrittstemperatur 80°C, Austrittstemperatur 50 - 52°C. Man erhält ein feines, gelbliches Pulver.

### Beispiel 3

10,95 g 1,2-Dipalmitoyl-3-sn-phosphatidylcholin, 4,6 g 1-Palmitoyl-2-oleoyl-3-sn-phosphatidylglycerolammonium, 418 mg Calciumchloriddihydrat und 750 mg Palmitinsäure werden in 330 ml 2-PropanoI/Wasser (85:15) bei 50°C gelöst und nach dem Abkühlen auf 30°C mit 620 ml einer Lösung von SP-C in IsopropanoI/Wasser (95 : 5, c = 484 mg/l) gemischt. Die resultierende Lösung wird in einem Laborsprühtrockner Büchi B 191 sprühgetrocknet. Sprühbedingungen: Trocknungsgas Stickstoff, Eintrittstemperatur 100°C, Austrittstemperatur 58 - 60°C. Man erhält ein farbloses Pulver.

### Beispiel 4

3,74g (5,1 mmol) 1,2-Dipalmitoyl-3-sn-phosphatidylcholin, 2,81 g (3,7 mmol) 1-Palmitoyl-2-oleoyl-3-sn-phosphatidylcholin, 2,90 g (3,9 mmol) 1,2-Dipalmitoylphosphatidyl-3-sn-phosphatidylglycerol-natrium, 234 mg Palmitinsäure und 279 mg (1,9 mmol) Catciumchloriddihydrat werden in 160 ml 2-Propanol/Wasser (85 :15) bei 50°C gelöst und nach dem Abkühlen auf 30°C mit 566 ml einer Lösung von SP-C in Isopropanol/Wasser (92 : 8, c = 330 mg/l) bei 30°C gemischt. Die resultierende Lösung wird in einem Laborsprühtrockner Büchi B 191 sprühgetrocknet. Sprühbedingungen: Trocknungsgas Stickstoff, Eintrittstemperatur 90°C, Austrittstemperatur 58 - 60°C. Man erhält ein farbloses Pulver.

### Beispiel 5

0,5 g RLLLLRLLLLRLLLLRLLLLR (R = Arg, L = Leu), 7,125 g 1,2-Dipalmitoyl-3-sn-phosphatidylcholin und 2,43 g 1-Palmitoyl-2-oleoyl-3-sn-phosphatidyl-glycerol-ammonium werden in 500 ml Chloroform/Methanol 1:1 unter Erwärmen auf 45°C gelöst und anschließend in einem Laborsprühtrockner Büchi B 191 sprühgetrocknet. Sprühbedingungen: Trocknungsgas Stickstoff, Eintrittstemperatur 85°C, Austrittstemperatur 55°C. Man erhält ein farbloses Pulver.

## Patentansprüche

1. Verfahren zur Herstellung einer pulverförmigen Lungensurfactant-Zubereitung enthaltend hydrophobe Lungensurfactant-Proteine, **dadurch gekennzeichnet, daß** man eine organische Lösung oder Suspension enthaltend hydrophobe Lungensurfactant-Proteine und gewünschtenfalls weitere Bestandteile einer Sprühtrocknung unterwirft.

2. Verfahren nach Patentanspruch 1, **dadurch gekennzeichnet, daß** als hydrophobe Lungensurfactant-Proteine SP-C und oder SP-B in der organischen Lösung oder Suspension vorliegen.

3. Verfahren nach Patentanspruch 2, **dadurch gekennzeichnet, daß** SP-C in der organischen Lösung oder Suspension vorliegt.

4. Verfahren nach Patentanspruch 1, **dadurch gekennzeichnet, daß** die organische Lösung oder Suspension 5 bis 15 Gew.-% Wasser enthält.

5. Verfahren nach Patentanspruch 1, **dadurch gekennzeichnet, daß** als weitere Bestandteile Phospholipide enthalten sind.

6. Verfahren nach Patentanspruch 1, **dadurch gekennzeichnet, daß** die Sprühtrocknung in einem erwärmten Gas durchgeführt wird.

7. Verfahren nach Patentanspruch 6, **dadurch gekennzeichnet, daß** als Gas Luft oder Stickstoff verwendet werden.

8. Verfahren nach Patentanspruch 6, **dadurch gekennzeichnet, daß** das Gas eine Eintrittstemperatur von 60 bis 200°C und eine Austrittstemperatur von 40 bis 80°C aufweist.

9. Verfahren nach Patentanspruch 7, **dadurch gekennzeichnet, daß** das Gas eine Eintrittstemperatur von 90 bis 150°C und eine Austrittstemperatur von 50 bis 70°C aufweist.

10. Pulverförmige Lungensurfactant-Zubereitungen erhältlich nach Verfahren nach den Patentansprüchen 1 bis 9.

11. Pulverförmige Lungensurfactant-Zubereitung nach Anspruch 10 mit einer Teilchengröße von 1 bis 5 µm.

12. Pulverförmige Lungensurfactant-Zubereitung nach Anspruch 10 oder 11 zur inhalativen Verabreichung.

13. Verwendung einer pulverförmigen Lungensurfactant-Zubereitung nach Anspruch 10 oder 11 zur Herstellung eines Arzneimittels für die inhalative Verabreichung.

## Claims

1. A process for producing a pulverulent pulmonary surfactant preparation containing hydrophobic pulmonary surfactant proteins, which comprises subjecting an organic solution or suspension containing hydrophobic pulmonary surfactant proteins and, if desired, further components to spray drying.

2. The process as claimed in claim 1, wherein the hydrophobic pulmonary surfactant proteins present in the organic solution or suspension are SP-C and/or SP-B.

3. The process as claimed in claim 2, wherein SP-C is present in the organic solution or suspension.

4. The process as claimed in claim 1, wherein the organic solution or suspension contains from 5 to 15% by weight of water.

5. The process as claimed in claim 1, wherein the further components are phospholipids.

6. The process as claimed in claim 1, wherein the spray drying is carried out in a heated gas.

7. The process as claimed in claim 6, wherein the gas used is air or nitrogen.

8. The process as claimed in claim 6, wherein the gas has an inlet temperature of from 60 to 200°C and an outlet temperature of from 40 to 80°C.

9. The process as claimed in claim 7, wherein the gas has an inlet temperature of from 90 to 150°C and an outlet temperature of from 50 to 70°C.

10. A pulverulent pulmonary surfactant preparation obtained by processes as claimed in claims 1 to 9.

11. A pulverulent pulmonary surfactant preparation as claimed in claim 10 having a particle size from 1 to 5 µm.

12. A pulverulent pulmonary surfactant preparation as claimed in claim 10 or 11 for administration by inhalation.

13. Use of a pulverulent pulmonary surfactant preparation as claimed in claim 10 or 11 for the manufacture of a medicament for administration by inhalation.

## Revendications

1. Procédé de fabrication d'une préparation de surfactant pulmonaire en poudre contenant des protéines hydrophobes du surfactant, **caractérisé par le fait que** l'on soumet une solution ou suspension organique contenant des protéines hydrophobes du surfactant et éventuellement d'autres composants, à un séchage par pulvérisation.

2. Procédé selon la revendication 1, **caractérisé par le fait que** les protéines hydrophobes du surfactant présentes dans la solution ou suspension organique sont la SP-C et/ou la SP-B.

3. Procédé selon la revendication 2, **caractérisé par le fait que** la solution ou suspension organique contient de la SP-C.

4. Procédé selon la revendication 1, **caractérisé par le fait que** la solution ou suspension organique contient de 5 à 15 % en poids d'eau.

5. Procédé selon la revendication 1, **caractérisé par le fait que** les autres composants comprennent des phospholipides.

6. Procédé selon la revendication 1, **caractérisé par le fait que** le séchage par pulvérisation se fait dans un gaz chauffé.

7. Procédé selon la revendication 6, **caractérisé par le fait que** l'on utilise en tant que gaz, de l'air ou de l'azote.

8. Procédé selon la revendication 6, **caractérisé par le fait que** la température d'entrée du gaz est comprise entre 60 et 200 °C, et que la température de sortie du gaz est comprise entre 40 et 80 °C.

9. Procédé selon la revendication 7, **caractérisé par le fait que** la température d'entrée du gaz est comprise entre 90 et 150 °C et que la température de sortie du gaz est comprise entre 50 et 70 °C.

10. Préparation de surfactant pulmonaire en poudre susceptible d'être obtenue selon un procédé conforme aux revendications 1 à 9.

11. Préparation de surfactant pulmonaire selon la revendication 10, ayant une taille de particules comprise entre 1 et 5 µm.

12. Préparation de surfactant pulmonaire en poudre selon la revendication 10 ou 11 destinée à une administration par inhalation.

13. Utilisation d'une préparation de surfactant pulmonaire en poudre selon la revendication 10 ou 11 pour la préparation d'un médicament destiné à être administré par inhalation.
